# EUROPÄISCHE PATENTSCHRIFT

(11) **EP 1 064 969 B1**
(45) Veröffentlichungstag und Bekanntmachung des Hinweises auf die Patenterteilung: **08.10.2003**
(21) Anmeldenummer: 99121840.5
(22) Anmeldetag: 04.11.1999
(51) Int. Cl.: A62B 7/06, F17C 9/02

(54) **Sauerstoffbeatmungsgerät**
Oxygen breathing apparatus
Appareil respiratoire à oxygène

(30) Priorität: 28.06.1999 DE 19929663
(43) Veröffentlichungstag der Anmeldung: 03.01.2001
(73) Patentinhaber: Blendien, Matthias, 78465 Konstanz (DE)
(72) Erfinder: Blendien, Helmut, 72160 Horb (DE)
(74) Vertreter: Klocke, Peter, Dipl.-Ing.

(56) Entgegenhaltungen:
- EP-A- 0 450 906
- GB-A- 509 105
- GB-A- 2 193 304
- US-A- 4 899 546

## Beschreibung

Die vorliegende Erfindung betrifft ein Sauerstoffbeatmungsgerät gemäß dem Oberbegriff des Anspruchs 1. Ein derartiges Gerät ist z.B. aus US-A-4 899 546 bekannt.

Derartige Sauerstoffbeatmungsgeräte sind allgemein bekannt. Sie bestehen aus einem Vorratsbehälter für den flüssigen Sauerstoff sowie einer auf dem Vorratsbehälter angeordneten Verdampfungseinrichtung, um den Sauerstoff in den gasförmigen Zustand zu überführen, damit er zur Beatmung eingesetzt werden kann. Um die entsprechende notwendige Durchflußmenge, insbesondere bei der Akutmedizin im Notfall zur Verfügung zu stellen, wird atmosphärischer Sauerstoff zugefügt. Bei der Langzeitherapie, bei der unter Umständen eine nicht so große Durchflußmenge benötigt wird, ist es jedoch zusätzlich erforderlich, den Sauerstoff anzufeuchten, um für den Patienten den gewünschten Sauerstoff zur Verfügung zu stellen. Die bisher bekannten Sauerstoffgeräte sind jedoch nicht geeignet, gleichzeitig als normales Sauerstoffgerät und in der Akutmedizin eingesetzt zu werden, da sie nicht in der Lage sind, den in dem Vorratsbehälter gespeicherten Sauerstoff mit einer Durchflußmenge von 1 bis 30 l pro Minute, je nach Bedarf, abzugeben.

Der vorliegenden Erfindung liegt daher die Aufgabe zugrunde, ein Sauerstoffbeatmungsgerät vorzuschlagen, das sowohl bei Notfällen als auch bei Nachbehandlungen mit niedrigerer Durchflußrate geeignet ist.

Diese Aufgabe wird erfindungsgemäß durch ein Sauerstoffbeatmungsgerät mit den Merkmalen des Anspruchs 1 gelöst. Weitere vorteilhafte Ausgestaltungen sind den Unteransprüchen zu entnehmen.

Danach weist das Sauerstoffbeatmungsgerät ein konzentrisches Doppelrohr auf, das zur Befüllung und Abnahme bis in den Bereich des Behälterboden hineinragt. Das äußere Rohr dient als Füllrohr und das innere Rohr als Steigrohr für den flüssigen Sauerstoff. Zusätzlich sind in dem Steigrohr Verwirbelungselemente angeordnet, die den aufsteigenden flüssigen Sauerstoff auf dem Weg zur Verdampfereinrichtung verwirbeln. Überraschenderweise wurde festgestellt, daß ein nicht laminar der Verdampfereinrichtung zuströmender flüssiger Sauerstoff wesentlich besser verdampft werden kann und dadurch dem Patienten 99,95 % Sauerstoff ohne weitere Zumischung eines anderen Sauerstoffes zugeführt werden kann. Die Sauerstoffzufuhr über die Maske kann dabei bis zu 30 l pro Minute betragen. Ein Liter Sauerstoff aus dem erfindungsgemäß ausgestalten Sauerstoffbeatmungsgerät ergibt 830 l atmungsfähiges Gas.

Gemäß einer weiteren bevorzugten Ausführungsform ist in dem Steigrohr ein weiteres mit Abstand zur Innenwand um eine Längsachse gedrehtes Seelenrohr angeordnet, das als Verwirbelungselement dient. Der der Verdampfungsrichtung zugeführte Sauerstoff wird damit sowohl im Inneren des Seelenrohres als auch in dem Zwischenraum zwischen dem Seelenrohr und dem Steigrohr geführt. Hierzu sind vorteilhafterweise in dem Steigrohr Abstandshalter angeordnet, die das Seelenrohr fixieren und gleichzeitig Durchflußöffnungen für zwischen dem Seelenrohr und dem Steigrohr aufsteigenden Sauerstoff aufweisen.

Gemäß einer bevorzugten Ausführungsform ist das Seelenrohr aus einem Kunststoffmaterial, vorzugsweise Polytetrafluorethylen (PTFE) hergestellt. Überraschenderweise wurde damit die besten Ergebnisse erzielt. Gemäß einer weiteren vorteilhaften Ausbildung ist das Seelenrohr mindestens zweimal um seine Längsachse gedreht. Diese Drehung reicht aus, den flüssigen Sauerstoff in ausreichende Unruhe auf seinem Weg in die Verdampfungseinrichtung zu versetzen. Besonders vorteilhaft wirkt sich diese zweimalige Drehung um die Längsachse aus, wenn die Drehrichtung des Seelenrohres entgegengesetzt zur Drehrichtung der Verdampferschlange in der Verdampfungseinrichtung ist. Der aus dem Seelenrohr wirbelnd austretende Sauerstoff wird dann durch die Verdampferschlange geführt, die über einen üblicherweise großen Durchmesser schraubenförmig geführt ist.

Nachfolgend wird die Erfindung anhand eines Ausführungsbeispiels näher erläutert. Es stellen dar:
- Figur 1: eine Schnittdarstellung durch das Sauerstoffbeatmungsgerät;
- Figur 2: eine schematische, vergrößerte Darstellung des oberen Bereichs des Sauerstoffbeatmungsgerätes;
- Figur 3: einen Querschnitt durch den oberen Bereich von Figur 2;
- Figur 4: ein vergrößerter Längsschnitt durch das Füll- und Steigrohr; und
- Figur 5: einen Querschnitt durch das Rohr gemäß Figur 4.

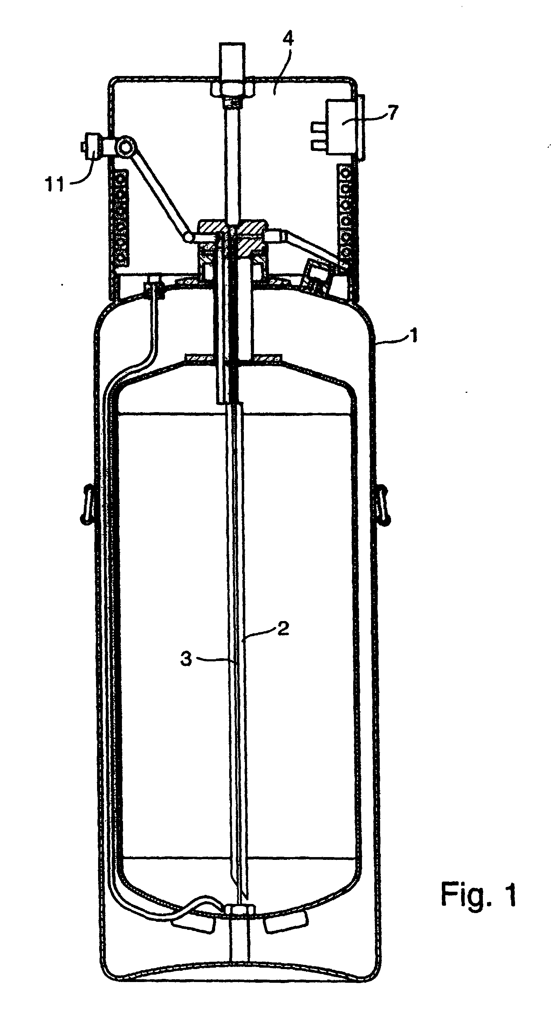

Bei dem in der Figur 1 dargestellten erfindungsgemäßen Sauerstoffbeatmungsgerät handelt es sich um ein stationäres sowie mobiles Sauerstoffbehältersystem, das ca. 47 l Flüssigsauerstoff in thermisch vakuumiertem, isolierten Doppeltank 1 als Vorratsbehälter für die häusliche

Sauerstoff-Langzeittherapie und zum Nachfüllen als mobiles, tragbares Sauerstoff-Behältersystem für die Notfallmedizin dient. Das Gerät ist für den vollen Einsatz im gesamten Rettungsgereich zu Lande, Wasser und in der Luft einsetzbar. Der Siedepunkt des flüssigen Sauerstoff beträgt -183° C. Ein Liter Flüssigsauerstoff ergibt ca. 860 Liter gasförmigen Sauerstoff. Der in dem Ausführungsbeispiel dargestellte Vorratsbehälter 1 ist ein Schutzbehälter, der den üblichen Vorschriften für derartige Behälter entspricht. Das Füllrohr 2 und das Steigrohr 3 reichen in den Vorratsbehälter bis in die Nähe des Behälterbodens, damit auch sämtlicher flüssiger Sauerstoff, der in den Vorratsbehälter enthalten ist, entnommen werden kann.

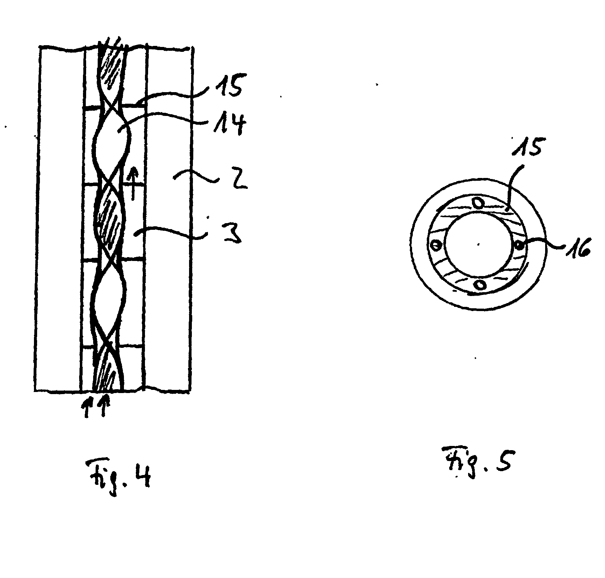

Der in dem Vorratsbehälter 1 befindliche Sauerstoff wird über ein in dem Füllrohr 2 angeordnetes Steigrohr 3 (Figur 4) in flüssige Form zu der Verdampfereinheit 4 aufgrund physikalischer Gesetze gepreßt und über entsprechende Ventile zu einem anschließbaren Durchflußmesser geführt, von dem über eine ebenfalls nicht dargestellte Verbindungsleitung zu dem Patienten führt. Der Patient wird mit einem reinen medizinischen Sauerstoff-Gasgemisch zu 100 % versorgt. Bis zu der Maske oder Nasenbrille des Patienten wird reiner Sauerstoff in einem Arbeitsbereich von 1 bis 20 l pro Minute angeboten.

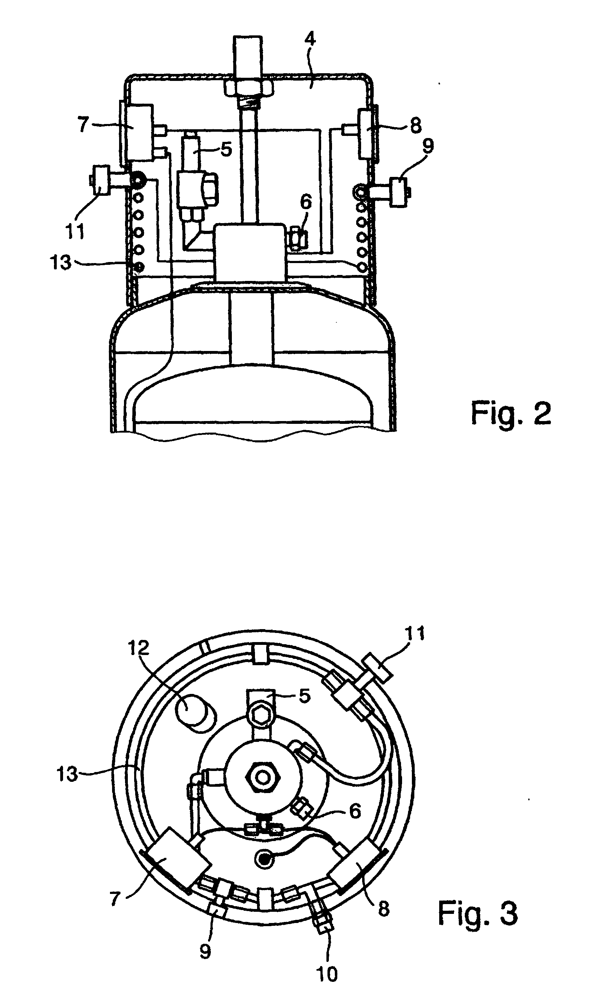

Die Verdampfereinheit 4 (Figur 2 und 3) ist mit für derartige Geräte bekannten Einrichtungen wie Sicherheitsventil 5, Oberströmventil 6, Inhaltsanzeige 7, Manometer 8, Sperrventil Gasentnahme 9, Anschluß für Durchflußmesser 10, Absperrventil Druckentlastung 11, Vakuumventil 12 und Verdampferschlange 13 versehen.

Figur 4 zeigt einen Längsschnitt durch das Füllrohr 2 mit dem im inneren angeordneten Steigrohr 3 sowie dem gewendelten Seelenrohr 14. Das Seelenrohr 14 ist, wie in Figur 5 gezeigt, über Abstandshalter in Form von Ringscheiben 15, die ihrerseits wiederum Öffnungen 16 für den Durchfluß des Sauerstoffs aufweisen, fixiert. Bei dem Steigrohr 3 handelt es sich um ein Edelstahlrohr und bei dem Seelenrohr 14 um ein PTEF-Rohr. Das Steigrohr 3 weist einen Innendurchmesser von 6 mm und das PTEF-Rohr einen Außendurchmesser von 4 mm auf. In einem Ausführungsbeispiel ist das Seelenrohr 14 mit einem Linksdrall versehen, während die anschließende Verdampferschlange 13 rechtsherum gebogen ist. In dem Ausführungsbeispiel weist das Seelenrohr eine Länge von 65 cm auf und ist auf diese Länge 2 mal um seine Achse gedreht.

## Patentansprüche

1. Sauerstoffbeatmungsgerät mit einem Vorratsbehälter in den ein Füllrohr und ein Abnahmerohr hineinragt sowie mit einer Verdampfungseinrichtung, die mit dem Abnahmerohr verbunden ist, **dadurch gekennzeichnet, daß** zur Befüllung und Abnahme bis in Bereich des Bodens des Vorratsbehälters (1) ein konzentrisches Doppelrohr in den Vorratsbehälter hineinragt, wobei das äußere Rohr (2) als Füllrohr und das innere Rohr (3) als Steigrohr für den flüssigen Sauerstoff dient, und in dem Steigrohr (3) Elemente (14, 15) zur Verwirbelung des aufsteigenden flüssigen Sauerstoffs angeordnet sind.

2. Sauerstoffbeatmungsgerät nach Anspruch 1, **dadurch gekennzeichnet, daß** in dem Steigrohr (3) ein weiteres mit Abstand zur Innenwand ein um seine Längsachse gedrehtes Seelenrohr (14) angeordnet ist, das als Verwirbelungselement dient.

3. Sauerstoffbeatmungsgerät nach Anspruch 2, **dadurch gekennzeichnet, daß** das Seelenrohr (14) aus einem Kunststoffmaterial, vorzugsweise Polytetrafluorethylen, hergestellt ist.

4. Sauerstoffbeatmungsgerät nach Anspruch 2 oder 3, **dadurch gekennzeichnet, daß** das Seelenrohr (14) mindestens 2 mal um seine Längsachse gedreht ist.

5. Sauerstoffbeatmungsgerät nach einem der vorangegangenen Ansprüche 2 bis 4, **dadurch gekennzeichnet, daß** die Drehrichtung des Seelenrohrs (14) entgegengesetzt zu der Drehrichtung der Verdampferschlange (13) in der Verdampfungsrichtung (4) ist.

6. Sauerstoffbeatmungsgerät nach einem der vorangegangenen Ansprüche 2 bis 5, **dadurch gekennzeichnet, daß** in dem Steigrohr (3) Abstandshalter (15) angeordnet sind, die das Seelenrohr fixieren und gleichzeitig Durchflußöffnungen (16) für zwischen dem Seelenrohr (14) und dem Steigrohr (3) aufsteigenden Sauerstoff aufweisen.

## Claims

1. Oxygen breathing apparatus, having a supply cylinder, into which a filler tube and a discharge tube protrude, and an evaporating means which is connected to the discharge tube, **characterised in that**, in order to effect filling and discharging in the region of the base of the supply cylinder (1), a concentric double tube protrudes into the supply cylinder, the outer tube (2) serving as the filler tube, and the inner tube (3) serving as the feedpipe for the liquid oxygen, and elements (14, 15) are disposed in the feedpipe (3) to swirl the rising liquid oxygen.

2. Oxygen breathing apparatus according to claim 1, **characterised in that** an additional cored tube (14), which is rotated about its longitudinal axis, is disposed in the feedpipe (3) and serves as a swirling element.

3. Oxygen breathing apparatus according to claim 2, **characterised in that** the cored tube (14) is produced from a plastics material, preferably polytetrafluoroethylene.

4. Oxygen breathing apparatus according to claim 2 or 3, **characterised in that** the cored tube (14) is rotated at least 2 times about its longitudinal axis.

5. Oxygen breathing apparatus according to one of the preceding claims 2 to 4, **characterised in that** the direction of rotation of the cored tube (14) is in the opposite direction to the direction of rotation of the evaporator coil (13) in the evaporating means (4).

6. Oxygen breathing apparatus according to one of the preceding claims 2 to 5, **characterised in that** spacer members (15) are disposed in the feedpipe (3), which spacer members secure the cored tube and simultaneously have throughflow apertures (16) for oxygen which rises between the cored tube (14) and the feedpipe (3).

## Revendications

1. Respirateur à oxygène comportant un réservoir de stockage dans lequel fait saillie un tube de remplissage et un tube de soutirage, ainsi qu'un dispositif de vaporisation, qui est relié au tube de soutirage, **caractérisé en ce que**, pour remplir ou soutirer jusque dans la zone du fond du réservoir de stockage (1), un double tube concentrique fait saillie dans le réservoir de stockage, le tube externe (2) servant de tube de remplissage et le tube interne (3) de tube montant pour l'oxygène liquide, et des éléments (14, 15) destinés à faire tourbillonner l'oxygène liquide montant (3) étant disposés dans le tube montant.

2. Respirateur à oxygène selon la revendication 1, **caractérisé en ce que** dans le tube montant (3) est disposé un autre tube d'âme (14) tourné autour de son axe longitudinal et placé selon un écart par rapport à la paroi interne, lequel tube sert d'élément induisant le tourbillon.

3. Respirateur à oxygène selon la revendication 2, **caractérisé en ce que** le tube d'âme (14) est fabriqué en un matériau plastique, de préférence du polytétrafluoroéthylène.

4. Respirateur à oxygène selon la revendication 2 ou 3, **caractérisé en ce que** le tube d'âme (14) est tourné au moins deux fois autour de son axe longitudinal.

5. Respirateur à oxygène selon l'une des revendications précédentes 2 à 4, **caractérisé en ce que** le sens de rotation du tube d'âme (14) est opposé au sens de rotation du serpentin évaporateur (13) dans le sens de la vaporisation (4).

6. Respirateur à oxygène selon l'une des revendications précédentes 2 à 5, **caractérisé en ce que** dans le tube montant (3) sont disposées des pièces d'écartement (15) qui fixent le tube d'âme et présentent simultanément des ouvertures de passage (16) pour l'oxygène montant entre le tube d'âme (14) et le tube montant (3).
